# EUROPÄISCHE PATENTSCHRIFT

(11) **EP 0 334 274 B1**
(45) Veröffentlichungstag und Bekanntmachung des Hinweises auf die Patenterteilung: **31.05.1995**
(21) Anmeldenummer: 89104998.3
(22) Anmeldetag: 21.03.1989
(51) Int. Cl.: A61N 5/02, A61H 1/00

(54) **Medizinisches Therapiegerät mit einer Liege mit einem unterhalb der Liegefläche angeordneten Diathermiegerät und einer Körperstreckeinrichtung**
Medical therapy device with a couch, a undercouch diathermic device and a body stretching device
Appareil de thérapie médicale avec une couchette dont le dessous comprend un appareil de diathermie et un dispositif d'étirement corporel

(30) Priorität: 21.03.1988 DE 8803793 U; 21.03.1988 DE 8803794 U
(43) Veröffentlichungstag der Anmeldung: 27.09.1989
(73) Patentinhaber: Spethmann, Jens, Dipl.-Ing., D-21339 Lüneburg (DE)
(72) Erfinder: Spethmann, Jens, Dipl.-Ing., D-21339 Lüneburg (DE)
(74) Vertreter: Richter, Werdermann & Gerbaulet

(56) Entgegenhaltungen:
- EP-A- 0 240 229
- WO-A-83/00620
- WO-A-84/02075
- DE-U- 8 218 927
- US-A- 4 546 766

## Beschreibung

Die Erfindung betrifft ein medizinisches Therapiegerät mit einer Liege mit einem unterhalb der Liegefläche in dem Liegengestell angeodneten Diathermiegerät, bestehend aus einem Magnetron, einem Strahler und einem Steuerteil, wobei die Liege eine Körperstreckeinrichtung aufweist, die aus einem an dem einen Ende der Liegefläche vorgesehenen, austauschbaren Patientengurt mit über mindestens eine an dem einen Ende der Liegefläche gelagerten Umlenkrolle geführtem und mit einer einen Steuerteil aufweisenden Spannvorrichtung verbundenem seilartigen Zugelement und aus einem an dem anderen Ende der Liegefläche vorgesehenen weiteren Patientengurt besteht, der über mindestens ein seilartiges Zugelement mit dem Liegengestell verbunden ist, wobei das Steuerteil eine Zugbegrenzungs-Umschaltung für die maximalen Kräfte der Halswirbelsäulen- und der Lendenwirbelsäulen-Extension aufweist, wobei die Spannvorrichtung als elektromotorische Zugeinrichtung, mit der eine gleichmäßige und betragsmäßig begrenzbare Zugkraft aufbringbar ist, ausgebildet ist.

Mikrowellen-Therapiegeräte (Diathermiegeräte) sind in den verschiedensten Ausführungsformen bekannt, so u. a. auch ein Mikrowellen-Therapiegerät mit einem Magnetron, einem Strahler und einem Steuerteil, bei dem das Gerät als Liege mit einem unterhalb der Liegefläche in dem Liegengestell in Liegenlängsrichtung verschieblich angeordneten, das Magnetron, den Strahler und den Steuerteil tragenden Schlitten ausgebildet ist. Bei der Anwendung solcher Geräte zu therapeutischen Zwecken hat es sich jedoch gezeigt, daß erst durch die gleichzeitige Anwendung einer weiteren therapeutischen Behandlungsform, nämlich der Extension des Patienten, d. h. z. B. Streckung der Wirbelsäule, die therapeutische Behandlung in Form von Erwärmung des Körpergewebes zu ihrem vollen, vorgesehenen Erfolg führt.

Es ist daher bereits ein medizinisches Therapiegerät vorgeschlagen worden, bei dem eine Liege mit einem unterhalb der Liegefläche in dem Liegengestell angeordneten und in Liegenlängsrichtung verschieblichen Schlitten versehen ist. Dabei ist auf dem Schlitten ein unterhalb der Liegefläche mit dem Schlitten verfahrbares Mikrowellen-Therapiegerät angeordnet, und die Liege weist eine Körperstreckeinrichtung auf, die aus einem an dem kopfseitigen Ende der Liegefläche vorgesehenen Patientengurt mit über mindestens eine an dem kopfseitigen Ende der Liegefläche gelagerte Umlenkrolle geführten und mit einer einen Steuerteil aufweisenden Spannvorrichtung verbundenen, seilartigen Zugelementen und aus einem an dem anderen Ende der Liegefläche vorgesehenen Patientengurt besteht. Hierbei ist eine elektro-mechanische Arbeitsweise der Spannvorrichtung vorgesehen, wobei die Erzeugung der nötigen Spannkräfte mittels Schrittmotor erfolgen soll (DE-U 82 18 927).

Ein derart ausgebildetes medizinisches Therapiegerät ermöglicht es, durch die Kombination der beiden therapeutischen Behandlungsformen, der Extension und der Erwärmung des Körpergewebes durch Hochfrequenzstrahlung, einen erhöhten therapeutischen Erfolg gegenüber der Einzelbehandlung zu erzielen, da sich die Wirkungen der Einzelbehandlungsformen gegenseitig verstärkend ergänzen. Bei diesem bekannten Therapiegerät hat es sich jedoch als nachteilig erwiesen, daß die LWS/HWS-Umschaltung, also die Umschaltung der lumbalen Therapie auf die cervicale Therapie von Hand durchgeführt werden muß. Wenn nämlich ein Patient bei der Cervical-Therapie mit den für die Lumbal-Therapie vorgesehenen Zugkräften beaufschlagt wird, kann es leicht zu Zerrungen und Überdehnungen kommen, die den gewünschten therapeutischen Erfolg nicht nur behindern, sondern den Behandlungserfolg völlig infrage stellen, da Schädigungen und Dauerschädigungen auftreten können.

Es ist daher Aufgabe der vorliegenden Erfindung, ein medizinisches Therapiegerät der eingangs genannten Art derart weiterzubilden, daß die Spannvorrichtung innerhalb der Liege möglichst raumsparend anordbar ist, daß die Vielzahl der bekannten Zug- und Umlenkelemente vermieden wird und die Spannvorrichtung trotz einfachsten Aufbaues eine sichere und gleichmäßige Aufbringung einer Zugkraft auf das zu beaufschlagende Zugelement sicherstellt. Gleichzeitig soll die Spannvorrichtung so steuerbar sein, daß die Aufbringung unerwünscht großer Zugkräfte, z. B. bei der Cervicalbehandlung, sicher vermeidbar ist, indem die Cervical-/Lumbalschaltung so automatisiert wird, daß Bedienungsfehler nicht auftreten können.

Diese Aufgabe wird durch die im Patentanspruch 1 gekennzeichneten Merkmale gelöst.

Erfindungsgemäß ist vorgesehen, daß das Steuerteil eine Schalt-Steckdose aufweist, in die ein schalt-Stecker zur Cervical-/Lumbal-Umschaltung bei gleichzeitiger Zugkraftbegrenzung einsteckbar ist, wobei der Schaltstecker mit einem Cervical-Gurt unlösbar verbunden ist. Es ist somit eine Schaltsteckdose vorgesehen, in der ein Schaltstecker zur Cervical-/Lumbal-Umschaltung sowie zur Cervical-Zugkraftbegrenzung einsteckbar ist, wobei der Schaltstecker mit dem Cervical-Gurt unlösbar verbunden ist, so daß verhindert wird, daß zur Cervicalbrechung zu hohe Zugkräfte angewandt werden.

Ein derart ausgebildeter Steuerteil ermöglicht es, automatisch und damit gegen menschliche Unzulänglichkeiten sicher zu vermeiden, daß bei der Durchführung einer Cervical-Therapie die für die Lumbal-Therapie vorgesehenen höheren Zugkräfte aufgebracht werden.

Gemäß einer bevorzugten Ausführungsform ist vorgesehen, daß die Spannvorrichtung von einer Antriebseinrichtung wie einem Elektromotor und einem an sich bekannten, aus einem Sonnenrad, einem Steg, drei Planetenrädern und einem Hohlrad bestehenden Planetengetriebe gebildet wird, die in einem Maschinengestell angeordnet sind, wobei das Sonnenrad über eine Antriebswelle mit der Antriebseinrichtung verbunden und über diese antreibbar ist, der Steg drehfest mit dem Maschinengestell verbunden ist und das Hohlrad drehbar und im Innenraum einer Seilwickeltrommel und mit dieser festverbunden angeordnet ist. Eine derart ausgebildete Spannvorrichtung ermöglilcht es, die Liege in ihrer Konzeption in vollem Umfang an die Bedürfnisse von Arztpraxen u. dgl. anzupassen, da der Raumbedarf der Spannvorrichtung äußerst klein und gleichzeitig die Extension besonders wirkungsvoll durchzuführen ist, da aufgrund der besonderen Gestaltung der Spannvorrichtung eine von Lastwechseln u. dgl. völlig unbeeinflußte Aufbringung einer gleichmäßigen Zugkraft möglich ist.

Gemäß einer weiteren bevorzugten Ausführungsform ist dabei vorgesehen, daß der Steg über eine Kraftmeßeinrichtung mit dem Maschinengestell verbunden ist. Auf diese Weise ist die insgesamt von der Spannvorrichtung aufgebrachte Zugkraft durch die Messung der entsprechenden Reaktionskraft überprüfbar, so daß die Messung immer mit der für derartige Geräte notwendigen Sicherheit durchführbar ist.

Ein derart ausgebildeter Steuerteil ermöglicht es, automatisch und damit gegen menschliche Unzulänglichkeiten sicher zu vermeiden, daß bei der Durchführung einer Cervical-Therapie die für die Lumbal-Therapie vorgesehenen höheren Zugkräfte aufgebracht werden.

Nach einer weiteren bevorzugten Ausführungsform ist dabei vorgesehen, daß der Schaltstecker aus einem Einsteckteil und einem Halterungsteil zur Halterung des Austausch-Patientengurtes besteht und daß das Steuerteil über eine flexible Leitung mit einem Not-/Aus-Schalter verbunden ist.

In der Zeichnung ist der Gegenstand der Erfindung dargestellt, und zwar zeigt
- Fig. 1: in einer Seitenansicht ein medizinisches Therapiegerät,
- Fig. 2: in einer Ansicht von oben das medizinische Therapiegerät und
- Fig. 3: in senkrecht geschnittener Darstellung eine Spannvorrichtung.

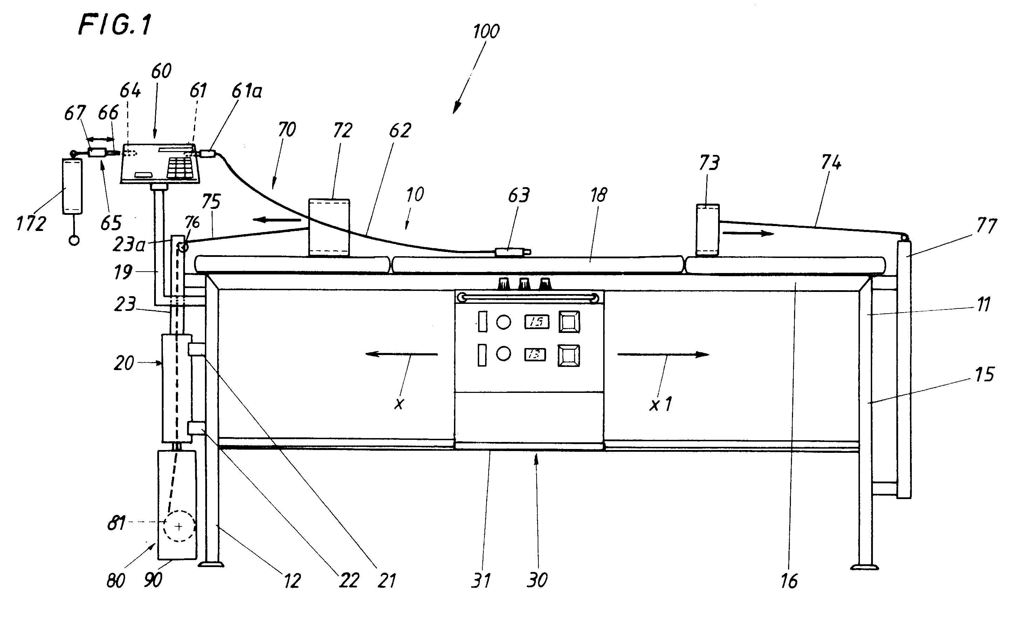

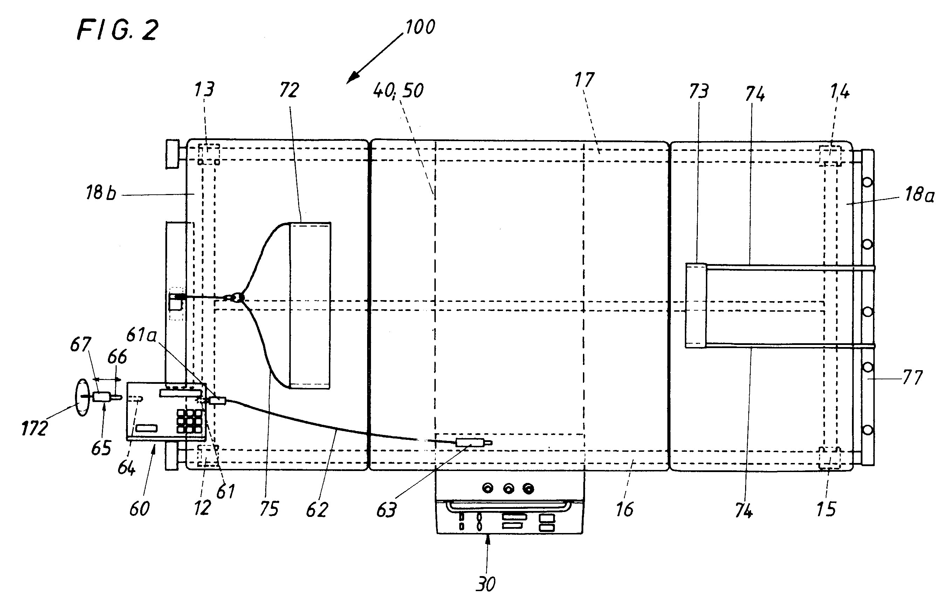

In Fig. 1 und 2 ist mit 100 ein medzinisches Therapiegerät bezeichnet, das aus einer Liege 10, einem Mikrowellen-Therapiegerät 30 und einer Körperstreckeinrichtung 70 besteht. Die Liege umfaßt ein Liegengestell 11 mit Standfüßen 12, 13, 14, 15 und einer vorzugsweise gepolsterten Liegefläche 18. Unterhalb der Liegefläche 18 ist in dem Liegengestell 11 eine in Liegenlängsrichtung verlaufende Führungsschiene angeordnet, auf der das Diathermiegerät verfahrbar eingehängt ist, das gehäuseartig ausgebildet ist und den in an sich bekannter Weise ausgebildeten Strahler 50, das Magnetron 40 und das Steuerteil 60 aufnimmt. Der Strahler 50 ist auf der Führungsschiene in Pfeilrichtung X, X1 verfahrbar.

Es kann auch vorgesehen sein, das Diathermiegerät hängend an einer Schiene zu lagern, welche an den Längsholmen 16, 17 des Liegengestells 11 befestigt ist.

Das auf einem Halterungsarm 19 am Zusatzaggregat angeordnete Steuerteil 60 nimmt die für die Betätigung des Therapiegerätes erforderlichen elektronischen Bauteile, Schaltuhren, Betätigungsknöpfe u. dgl. auf. Mit dem Steuerteil 60 wird die Spanneinrichtung 80 gesteuert, wobei in der Steuereinrichtung 60 eine Steckdose 61 vorgesehen ist, in die das steckerartige Ende 61a einer Leitung 62 einsteckbar ist, an deren anderem Ende ein Betätigungsschalter 63 angeordnet ist, mit dem ein Not-Stopp-Signal bzw. ein Steuersignal auslösbar ist.

Die Steuereinrichtung 60 ist mit einer Schaltsteckdose 64 versehen, in die ein Schaltstecker 65 frei einsteckbar ist. Der Schaltstecker 65 besteht dabei aus einem Einsteckteil 66, das die gewünschte Umschaltung bewirkt und aus einem Halterungsteil, mit dem der Cervical-Gurt 172 unlösbar verbunden ist. Bei dem Cervical-Gurt 172 handelt es sich um einen Gurt für die Cervical-Extension, der am Kopf oder am Hals befestigbar ist. In der Schaltsteckdose 64 ist dabei eine in der Zeichnung nicht dargestellte Sperre vorgesehen, die bei Herausziehen des Einsteckteiles 66 ausgelöst wird und innerhalb der Steuerung im Steuerteil 60 automatisch die Zugkräfte begrenzt. Von der Spannvorrichtung 80 werden dann nur noch diejenigen Zugkräfte aufgebracht, die bei der Halswirbelextension gewünscht und zumutbar sind. Wenn dann der Halswirbelgurt 172 bei Durchführung einer neuen weiteren Behandlung nicht mehr benötigt wird, dann wird der Schaltstecker 65 in die Schaltsteckdose 64 eingeteckt, wodurch der Cervical-Gurt am Steuerteil 60 gehaltert ist. Durch das Einstecken des Einsteckteiles 66 wird die Sperre betätigt, so daß die für eine Lendenwirbelbehandlung notwendigen höheren Zugkräfte über das Steuerteil 60 freigegeben werden, die dann von der Spannvorrichtung 80 aufgebracht werden.

Sofern hier von der Bedienperson versehentlich bei einer Lumbal-Therapie der Schaltstecker 65 nicht ordnungsgemäß in die Schaltsteckdose 64 eingesteckt wird, werden von der Spannvorrichtung 80 lediglich die zur Cervical-Therapie vorgesehenen niedrigeren Zugkräfte aufgebracht. Eine Gefährdung für den Patienten ist hierdurch nicht gegeben.

Durch die weitere Sicherung über die Leitung 62 und den Betätigungsschalter 63 ist auch eine momentane Überbeanspruchung des Patienten bei korrekt vorgegebenen Zugkräften ausgeschlossen, da dieser bei Auftreten eines nicht gewünschten Spannungs- und/oder Schmerzzustandes ein Stopp-Signal auslösen kann, bei dem ein automatischer Entspannungsvorgang auftritt.

Auf der Liegefläche 18 der Liege 10 sind obenseitig und in den beiden Liegeflächenendbereichen 18a, 18b gurtartige Körperhalterungen frei gelagert, nämlich Patientengurte 72, 73, die an der therapeutisch zu behandelnden, auf der Liegefläche 18 liegenden Behandlungsperson an den hierzu geeigneten Körperpartien, wie Hals, Brustkorb, Unterschenkel od. dgl. angebracht werden. Die Patientengurte 72, 73, die entsprechend größen- und längenveränderbar ausgebildet sind, sind mit entsprechenden seilartigen Zugelementen 74, 75 lösbar verbunden. Das seilartige Zugelement 74, das mit dem Patientengurt 73 verbunden ist, ist dabei fest über eine Haltestange 77 mit dem Liegengestell 11 verbunden, so daß eine auf den Patientengurt 73 aufgebrachte Zugkraft über das Liegengestell 11 abgestützt wird. Der Patientengurt 72 ist mit dem Zugelement 75 verbunden, das über die Umlenkrolle 76 mit der Spannvorrichtung 80 verbunden ist.

Die Spannvorrichtung 80 ist an einem Tragrahmen 20 angeordnet, wobei unterhalb der Liegefläche 18 an dem Liegengestell 11 zwischen den Standfüßen 12, 13 in Liegenquerrichtung verlaufende horizontale Führungsschienen 21, 22 parallel zueinander und in einem Abstand voneinander verlaufend angeordnet sind, auf denen das Traggestell 20, das gehäuseartig ausgebildet ist, quer verfahrbar ist. An dem Traggestell 20 ist ein höhenverstellbarer, nach oben auskragender Tragarm 23 angeordnet, der ein Hohlprofil aufweist, in dessen Innenraum das Zugelement 75 der Spannvorrichtung 80 zugeführt wird und an dessen auskragendem Ende 23a die Umlenkrolle 76 drehbar angeordnet ist. Somit ist die Applizierung der Zugkraft aus unterschiedlichen räumlichen Richtungen möglich.

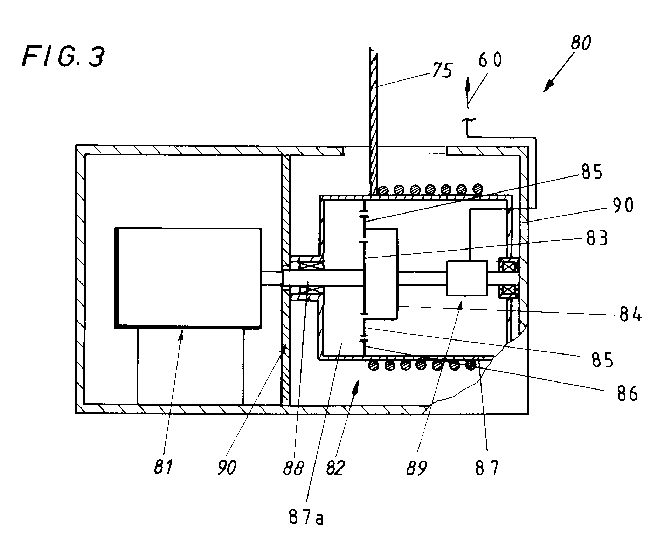

Die Spannvorrichtung 80, die in Fig. 3 näher dargestellt ist, besteht aus einem an dem Tragrahmen 20 angeordneten Maschinengestell 90, in dem eine Antriebseinrichtung 81 wie ein Elektromotor und ein Stirnradplanetengetriebe 82 angeordnet ist. Das Stirnradplanetengetriebe 82 besteht dabei aus einem Sonnenrad 83, einem Steg 84, drei Planetenrädern 85 und einem Hohlrad 86, die in an sich bekannter Weise zueinander angeordnet sind. Die Antriebseinrichtung 81, die fest in dem Maschinengestell 90 gehaltert ist, ist mit ihrer Antriebswelle 88 mit dem Sonnenrad 83 verbunden und beaufschlagt somit den Eingang des Planetengetriebes 82. Der Steg 84 ist über eine elektromechanische Kraftmeßeinrichtung 89 mit dem Maschinengestell 90 verbunden, wobei der Steg 84 insofern drehfest mit dem Maschinengestell 90 verbunden ist, als nur eine zur Ausbildung des Meßweges der Kraftmeßeinrichtung 89 vorzusehende kleine Drehbewegung möglich ist. Das bei konventionellen Anordnungen teilweise drehfest angeordnete Hohlrad 86 ist dabei in dem Maschinengestell 90 frei drehbar angeordnet. Es ist dabei im Innenraum 87a einer ebenfalls frei drehbar angeordneten Seilwickeltrommel 87 mit dieser fest verbunden, so daß eine Drehung der Antriebswelle 88 von dem Sonnenrad 83 über die Planetenräder 85 auf das Hohlrad 86 übertragen wird, das seinerseits die Seilwickeltrommel 87 zu einer Drehbewegung antreibt. Dabei wird das Seil 75 entsprechend auf- bzw. bei Eingang eines entsprechenden Meßsignals von der Kraftmeßeinrichtung 89 und einer dadurch bewirkten Umschaltung der Antriebseinrichtung 81 abgewickelt.

Die Antriebseinrichtung 81 kann dabei auch ein Schaltwerksgetriebe enthalten oder mit einem Schrittmotor versehen sein. Bei Vorgabe einer entsprechenden Spannkraft über die Steuereinrichtung 60 wird von der Spannvorrichtung 80 immer die gewünschte und für eine Extension der Behandlungsperson notwendige Spannkraft erzeugt, auf das Zugelement 75 übertragen und über die Kraftmeßeinrichtung 89 der Höhe nach kontrolliert. Auf diese Weise ist dann eine gleichmäßige Körperextension möglich.

Die Erfindung ist nicht beschränkt auf die voranstehend beschriebenen Ausführungsformen. Eine andere Anordnung des Steuerteils liegt ebenso im Rahmen der Erfindung wie eine Anordnung der Schaltsteckdose beispielsweise in den Zuleitungen zum Steuerteil oder in der Zeichnung nicht dargestellten Verbindungsleitung zwischen Steuerteil und Spannvorrichtung.

## Patentansprüche

1. Medizinisches Therapiegerät (100) mit einer Liege (10) mit einem unterhalb der Liegefläche (18) in dem Liegengestell (11) angeordneten Diathermiegerät, bestehend aus einem Magnetron (40) und einem Strahler (50), und einem Steuerteil (60), wobei die Liege eine Körperstreckeinrichtung (70) aufweist, die aus einem an dem einen Ende (18b) der Liegefläche (18) vorgesehenen, austauschbaren Patientengurt (72) mit über mindestens eine an dem einen Ende (18b) der Liegefläche (18) gelagerten Umlenkrolle (76) geführtem und mit einer einen Steuerteil aufweisenden Spannvorrichtung (80) verbundenem seilartigen Zugelement (75) und aus einem an dem anderen Ende (18a) der Liegefläche vorgesehenen weiteren Patientengurt (73) besteht, der über mindestens ein seilartiges Zugelement (74) mit dem Liegengestell (11) verbunden ist, wobei das Steuerteil (60) eine Zugbegrenzungs-Umschaltung für die maximalen Kräfte der Halswirbelsäulen- und der Lendenwirbelsäulen-Extension aufweist, wobei die Spannvorrichtung (80) als elektromotorische Zugeinrichtung, mit der eine gleichmäßige und betragsmäßig begrenzbare Zugkraft aufbringbar ist, ausgebildet ist, dadurch gekennzeichnet,
daß das Steuerteil (60) eine Schalt-Steckdose (64) aufweist, in die ein Schaltstecker (65) zur Cervical-/Lumbal-Umschaltung einsteckbar ist, wobei der Schaltstecker (65) mit einem Cervical-Gurt (172) unlösbar verbunden ist.

2. Medizinisches Therapiegerät nach Anspruch 1, dadurch gekennzeichnet, daß die Spannvorrichtung (80) von einer Antriebseinrichtung (81) wie einem Elektromotor und einem an sich bekannten, aus einem Sonnenrad (83), einem Steg (84), drei Planetenrädern (85) und einem Hohlrad (86) bestehenden Planetengetriebe (82) gebildet wird, die in einem Maschinengestell (90) angeordnet sind, wobei das Sonnenrad (83) über eine Antriebswelle (88) mit der Antriebseinrichtung (81) verbunden und über diese antreibbar ist, der Steg (84) drehfest mit dem Maschinengestell (90) verbunden ist und das Hohlrad (86) drehbar und im Innenraum (87a) einer Seilwickeltrommel (87) und mit dieser festverbunden angeordnet ist.

3. Medizinisches Therapiegerät nach Anspruch 2, dadurch gekennzeichnet, daß der Steg (84) über eine Kraftmeßeinrichtung (89) mit dem Maschinengestell (90) verbunden ist.

4. Medizinisches Therapiegerät nach einem der Ansprüche 1 bis 3, dadurch gekennzeichnet, daß der Schaltstekker (65) aus einem Einsteckteil (66) und einem Halterungsteil (67) zur Halterung des Austausch-Patientengurtes (172) besteht.

5. Medizinisches Therapiegerät nach einem der Ansprüche 1 bis 4, dadurch gekennzeichnet, daß das Steuerteil (60) über eine flexible Leitung (62) mit einem Not-/Aus-Schalter (63) verbunden ist.

## Claims

1. Medical therapy unit (100) comprising a couch (10) with a diathermy facility disposed underneath the coach surface (18) in the couch frame (11), comprising a magnetron (40) and a radiator (50) as well as a control member (60), wherein the couch is provided with a body stretching appliance (70), which is comprised of an interchangeable patient's belt (72) provided on one end (18b) of the couch surface (18) with a cable-like traction element (75) led across at least one guide roller (76) mounted on one of the ends of the couch surface and connected to a tightening device (80) possessing a control member (60) and of a further patient's belt (73) provided on the other end (18a) of the coach surface which, by means of at least one cable-like traction element (74) is connected to the couch frame (11), in which the control member (60) possesses a traction-limiting changeover switching means for the maximal forces of the cervical spine and lumbar spine extension, in which case the tightening device (80) is constructed in the form of an electromotive traction apparatus, with the aid of which a uniform and magnitude-wise limiting traction can be applied,
characterized in that
the control member (60) possesses a switching socket (64), into which a switching connector (65) for the cervical/lumbar changeover switching means is insertable, while the switching connector (65) is nnmdetachably connected to a cervical strap (172).

2. Medical therapy unit according to Claim 1,
characterized in that
the tightening device (80) is constituted of a driving means (81), such as an electromotor and a planetary gearing known per se and comprising a sun wheel (83), a web (84), three planet pinions (85) and one hollow wheel (86), which are disposed in a bedplate (90), wherein the sun wheel (83) is connected with the driving means (81) with the aid of a drive shaft (88) and can be driven by means of the same, the web (84) being connected in a torsionally resistant manner with the bedplate (90) and the hollow wheel (86) being disposed so as to be rotatable and, in the interior (87a) of a cable drum (87),being rigidly connected to the same.

3. Medical therapy unit according to Claim 2,
characterized in that
the web (84) is connected with the bedplate (90) by means of a dynamometric device (89).

4. Medical therapy unit according to any of Claims 1 to 3,
characterized in that
the switching connector (65) is comprised of a plug-in type portion (66) and a mounting portion (67) for mounting the interchangeable patient's belt (172).

5. Medical therapy unit according to any of Claims 1 to 4,
characterized in that
the control member (60) is connected to an emergency cut-out switch (63).

## Revendications

1. Appareil de thérapie médicale (100) avec un lit (10) avec un appareil de diathermie, placé sous la couchette (18) dans le bâti du lit (11), constitué par un magnétron (40), un émetteur de rayonnement (50) et une partie de commande (60), le lit présentant un dispositif d'extension du corps (70) qui est constitué par une ceinture pour patient (72) interchangeable, prévue à l'une des extrémités (18b) de la couchette (18), avec un élément de traction du type câble (75) guidé par au moins une poulie de renvoi (76) positionnée à l'une des extrémités (18b) de la couchette (18) et relié à un dispositif de tension (80) qui présente une partie de commande et par une autre ceinture pour patient (73), prévue à l'autre extrémité (18a) de la couchette, qui est reliée par au moins un élément de traction du type câble (74) au bâti du lit (11), la partie de commande (60) présentant une inversion de la limitation de la traction pour les forces maximales de l'extension de la colonne vertébrale cervicale et de la colonne verticale lombaire, le dispositif de tension (80) étant configuré comme un dispositif de traction électromotrice avec lequel une force de traction régulière d'une valeur qui peut être limitée peut être exercée, **caractérisé en ce**
que la partie de commande (60) présente une prise de commutation (64) dans laquelle une fiche de commutation (65) peut être insérée pour la commutation thérapie cervicale/thérapie lombaire, la fiche de commutation (65) étant reliée de manière inamovible à la ceinture cervicale (172).

2. Appareil de thérapie médicale selon la revendication 1, **caractérisé en ce** que le dispositif de tension (80) est formé par un dispositif d'entraînement (81) comme un moteur électrique et un engrenage planétaire (82) connu en soi, constitué par une roue solaire (83), une entretoise (84), trois roues planétaires (85) et une couronne de train planétaire (86) qui sont placées dans un bâti de machine (90), la roue solaire (83) étant reliée par un arbre moteur (88) au dispositif d'entraînement (81) et pouvant être entraînée par celui-ci, l'entretoise (84) étant reliée de manière résistante à la torsion au bâti de machine (90) et la couronne de train planétaire (86) étant rotative et placée dans l'espace intérieur (87a) d'un rouleau enrouleur de câble (87) en étant reliée de manière fixe à celui-ci.

3. Appareil de thérapie médicale selon la revendication 2, **caractérisé en ce** que l'entretoise (84) est reliée par l'intermédiaire d'un dispositif dynamométrique (89) au bâti de machine (90).

4. Appareil de thérapie médicale selon l'une des revendications 1 à 3, **caractérisé en ce** que la fiche de commutation (65) est constituée par une partie à insérer (66) et une partie de support (67) pour supporter la ceinture interchangeable pour patient (172).

5. Appareil de thérapie médicale selon l'une des revendications 1 à 4, **caractérisé en ce** que la partie de commande (60) est reliée par une conduite flexible (62) à un commutateur d'arrêt d'urgence (63).
